# EUROPÄISCHE PATENTSCHRIFT

(11) **EP 2 161 022 B1**
(45) Veröffentlichungstag und Bekanntmachung des Hinweises auf die Patenterteilung: **10.10.2012**
(21) Anmeldenummer: 09010077.7
(22) Anmeldetag: 05.08.2009
(51) Int. Cl.: A61K 31/19, A61K 9/16, A61K 9/20

(54) **Herstellung von Phosphatbindern und auf diese Weise hergestellte Phosphatbinder**
Production of phosphate connectors and phosphate connectors produced according to the method
Fabrication de liants de phosphate et liants de phosphate ainsi fabriqués

(30) Priorität: 05.09.2008 DE 102008045831; 07.10.2008 DE 102008051572
(43) Veröffentlichungstag der Anmeldung: 10.03.2010
(73) Patentinhaber: Maria Clementine Martin Klosterfrau Vertriebsgesellschaft mbH, 50670 Köln (DE)
(72) Erfinder: Vestweber, Anna-Maria, Dr. med., 51399 Burscheid (DE); Schlüter, Andreas F., 29439 Lüchow (DE)
(74) Vertreter: Gesthuysen, von Rohr & Eggert

(56) Entgegenhaltungen:
- WO-A-99/01121
- US-A- 2 731 862
- US-A1- 2007 128 271

## Beschreibung

Die vorliegende Erfindung betrifft die Herstellung von Phosphatbindern und auf diese Weise hergestellte Phosphatbinder, welche sich insbesondere für die Formulierung bzw. Produktion von Arzneimitteln zur Behandlung von mit renalen Dysfunktionen, insbesondere Niereninsuffizienz (z. B. infolge von Diabetes), einhergehenden Erkrankungen bzw. Störungen, wie Hyperphosphatämie und/oder Hypercalcämie, verbunden mit den hierdurch bedingten Erkrankungen (z. B. renale Osteodystrophie, sekundärer Hyperparathyroidismus etc.), eignet.

Insbesondere betrifft die vorliegende Erfindung ein Verfahren zur Herstellung einer Zusammensetzung, welche sich zur prophylaktischen bzw. therapeutischen Behandlung der oben genannten Erkrankungen bzw. Störungen eignet, wobei die Zusammensetzung eine Kombination von Calciumacetat einerseits und Magnesiumsalz andererseits aufweist.

Zudem betrifft die vorliegende Erfindung eine Zusammensetzung als solche auf Basis einer Kombination von Calciumacetat einerseits und Magnesiumsalz andererseits sowie die Verwendung dieser Zusammensetzung zur prophylaktischen bzw. therapeutischen Behandlung der oben genannten Erkrankungen bzw. Störungen.

Weiterhin betrifft die vorliegende Erfindung die Verwendung einer Kombination von Calciumacetat einerseits und Magnesiumsalz andererseits zur Herstellung einer Zusammensetzung zur Behandlung der zuvor beschriebenen Erkrankungen bzw. Störungen.

Darüber hinaus betrifft die vorliegende Erfindung ein Verfahren zur Herstellung von partikulärem Calciumacetat und das auf diese Weise hergestellte partikuläre Calciumacetat als solches.

Schließlich betrifft die vorliegende Erfindung ein Arzneimittel, welches sich insbesondere zur prophylaktischen bzw. therapeutischen Behandlung der zuvor genannten Erkrankungen bzw. Störungen eignet und welches partikuläres Calciumacetat enthält.

Eine Fehlfunktion bzw. Störung der natürlichen Funktion der Nieren, wie oftmals in chronischer Form vorliegendes Nierenversagen bzw. Niereninsuffizienz ist ein häufig auftretendes Krankheitsbild. So leiden weltweit mehr als 500 Millionen Menschen an einer chronischen Nierenkrankheit, wobei es bei etwa 1,5 Millionen Menschen erforderlich ist, diese Fehlfunktion durch eine regelmäßig durchzuführende Dialyse zu kompensieren.

Die häufigste Ursache für eine Nierenerkrankung sind Entzündungen der Filtereinheiten der Niere, was als sogenannte Glomerulonephritis bezeichnet wird. Weitere Ursachen für Nierenerkrankungen bzw. Nierenschäden liegen in einer chronischen Zuckerkrankheit, in einer als Pyelonephritis bezeichneten Nierenbeckenentzündung oder in einer Schädigung der Nieren durch Medikamente. Die Gefahr von Nierenerkrankungen bis hin zur Niereninsuffizienz besteht auch bei Personen mit Hypertonie und Menschen mit Übergewicht. Auch Raucher weisen ein erhöhtes Risiko auf, Nierenerkrankungen zu entwickeln bzw. an Niereninsuffizienz zu erkranken.

Fehlfunktionen der Nieren gehen häufig mit gravierenden gesundheitlichen Problemen einher und stellen eine große Belastung für die davon betroffenen Personen dar, insbesondere da die Niere für zahlreiche physiologische Aufgaben, wie die Entfernung von Stoffwechselprodukten aus dem Blut, die Regulation des Flüssigkeits- und Elektrolythaushaltes sowie die spezifische Produktion und Abgabe von Hormonen in das Blut, verantwortlich ist.

So sind die Nieren auch für das Gleichgewicht des Phosphathaushaltes essentiell. Bei einer terminalen Niereninsuffizienz ist der Phosphathaushalt oftmals erheblich gestört insofern, als bei unter Niereninsuffizienz leidenden Personen oftmals eine sogenannte Hyperphosphatämie vorliegt. Die Erhöhung der Phosphatkonzentration im Blut der betroffenen Personen ist dabei durch eine verminderte Ausscheidung von Phosphat durch die Nieren begründet. Die Hyperphosphatämie kann dabei Folgebeschwerden, wie Juckreiz (Pruritus) oder eine gerötete Augenbindehaut (Conjunctiva), verursachen. Gemeinsam mit einer Hypercalcämie kann eine Hyperphosphatämie zudem die Erregbarkeit der Muskeln und Nerven steigern, so daß es zu einem tetanischen Syndrom mit Fühlstörungen und Krämpfen bis hin zu einem tetanischen Anfall kommen kann.

Eine Hyperphosphatämie geht bei unter Niereninsuffizienz leidenden Personen oftmals mit einer Hypercalcämie, also einer Erhöhung der Calciumkonzentration bzw. des Calciumspiegels im Blut, einher. Die diesbezüglichen Zusammenhänge sind darin zu sehen, daß der die Phosphat- bzw. Calciumkonzentration im Blut regulierende Hormonkreis gestört ist: Im allgemeinen ist das in der Nebenschilddrüse gebildete Parathormon (PTH) für eine Regulation von Phosphat und Calcium im Blut verantwortlich, indem es über Veränderungen der Nierenfunktion die Ausscheidung von Phosphat bzw. Calcium reguliert. Bei Personen mit Niereninsuffizienz kann eine entsprechende Phosphatausscheidung jedoch nicht in ausreichendem Maße bewerkstelligt werden, und auch im Rahmen einer Dialyse ist die Phosphatentfernung aus dem Blut mitunter eingeschränkt. Als Folge der erhöhten Phosphatkonzentration im Blut wird jedoch wiederum die vermehrte Bildung und Freisetzung des Parathormons induziert. Ein Anstieg der Konzentration des Parathormons im Blut bewirkt insbesondere durch Aktivierung von für die Resorption von Knochensubstanz verantwortlichen Osteoklasten eine Calciumfreisetzung aus den Knochen, so daß der Calciumspiegel im Blut steigt. Entsprechend liegt bei unter Niereninsuffizienz leidenden Patienten bzw. Dialysepatienten neben einer Hyperphosphatämie häufig auch eine Hypercalcämie vor. Eine erhöhte Calciumkonzentration im Blut kann jedoch zur Bildung von unerwünschten Ablagerungen in den Blutgefäßen und anderen Geweben führen, so daß auch die Gefahr für Erkrankungen des Herz/Kreislauf-Systems zunimmt.

Eine Hyperphosphatämie kann insbesondere gemeinsam mit einer Hypercalcämie zu Weichteilverkalkungen führen. So sind Verkalkungen der Augenlinsen, Verkalkungen im Gehirn, einhergehend mit Bewegungsstörungen, Sprachstörungen und einer sich langsam entwickelnden Demenz, möglich.

Vor diesem Hintergrund besteht bei unter Niereninsuffizienz bzw. Nierenversagen leidenden Personen bzw. Dialysepatienten die Notwendigkeit, therapeutische Maßnahmen hinsichtlich der Regulation des Elektrolythaushaltes, insbesondere im Hinblick auf die zuvor beschriebene Hyperphosphatämie bzw. Hypercalcämie, zu realisieren. In diesem Zusammenhang werden im Stand der Technik insbesondere bei Dialysepatienten zur Therapie der als Folge der Grundkrankheit, nämlich Niereninsuffizienz, auftretenden Hyperphosphatämie sogenannte Phosphatbinder eingesetzt, welche zu einer Normalisierung der Phosphatkonzentration bzw, des Phosphatspiegels im Blut führen sollen. Ein therapeutischer Ansatz, welcher auf eine Behandlung der Hyperphosphatämie abzielt, führt dabei aufgrund der zuvor beschriebenen Regelmechanismus auf Basis des Parathormons auch zu einer Regulation des Calciumspiegels und damit zu einer Verbesserung der Hypercalcämie.

In diesem Zusammenhang sind im Stand der Technik verschiedene therapeutische Ansätze bekannt, bei welchen Phosphatbinder zur Behandlung der Hyperphosphatämie zum Einsatz kommen:

Eine erste Gruppe von Arzneimitteln stellen sogenannte aluminiumhaltige Phosphatbinder dar, welche effektiv Phosphate der Nahrung im Darm binden können. Nachteilig hierbei ist jedoch, daß Aluminium mitunter zu schweren Nebenwirkungen, wie Knochenerkrankungen, Anämie, Gehirnschäden bis hin zu Demenzerkrankungen mit Denk- und Wahrnehmungsstörungen und Merkschwäche, führen kann. Dies gilt insbesondere dann, wenn aufgrund der Niereninsuffizienz über den Darm resorbiertes Aluminium nicht mehr in effektiver Weise aus dem Körper entfernt werden kann. Demnach sind Phosphatbinder auf Aluminiumbasis nicht das Mittel der Wahl.

Ein zweiter therapeutischer Ansatz besteht in der Verabreichung von Formulierungen, welche den Wirkstoff Sevelamer, insbesondere Sevelamerhydrochlorid, enthalten. Die Substanz bindet im Darm Phosphate und Cholesterin, so daß neben dem Phosphatspiegel auch der Cholesterinspiegel gesenkt werden kann. Jedoch sind Medikamente auf Basis von Sevelamer als aktive Wirkkomponente hinsichtlich der Wirkung auf eine Verminderung des Phosphatspiegels im Blut nicht immer ausreichend effektiv.

Weiterhin ist es im Stand der Technik vorgesehen, zur Behandlung der Hyperphosphatämie Medikamente einzusetzen, welche als Phosphatbinder Calciumacetat enthalten. Calciumacetat ist hinsichtlich der Verminderung des Phosphatspiegels wirksamer als Sevelamer und weist darüber hinaus nicht die mit Aluminium einhergehenden Nebenwirkungen auf. Zur Verminderung eines übermäßigen Anstieges der Calciumkonzentration im Blut ist es dabei im Stand der Technik vorgesehen, Calciumacetat im Rahmen von Kombinationspräparaten gemeinsam mit Magnesiumcarbonat zu verabreichen. Magnesiumcarbonat fungiert dabei als Antagonist hinsichtlich der Aufnahme von Calcium in das Blut, was einer Hypercalcämie entgegenwirkt. Gleichzeitig führt die Verwendung von Magnesium zu einer Senkung des Parathormonspiegels, was sich gleichermaßen zu einer Verbesserung bzw. Linderung der Hyperphosphatämie und der Hypercalcämie führt.

Nachteilig bei den im Stand der Technik bekannten Kombinationspräparaten auf Basis von Calciumacetat und Magnesiumcarbonat ist jedoch, daß die Zusammensetzungen als solche oftmals keine ausreichende chemische Stabilität aufweisen, da die Wirkstoffkomponenten Calciumacetat und Magnesiumcarbonat unter Freisetzung von Essigsäure und Kohlendioxid zersetzt werden können.

Im Stand der Technik ist es dabei vorgesehen, die Calciumacetat-Komponente mittels Hordentrocknung bereitzustellen, wobei Calciumacetat enthaltendes Gut auf Bleche dünn aufgetragen bzw. geschichtet und von erwärmter Luft überströmt wird, gefolgt von einem anschließenden Sieben bzw. Klassieren. Die zur Bereitstellung der Calciumacetat-Komponente im Stand der Technik vorgesehene Hordentrocknung weist jedoch gravierende Nachteile auf, welche sich auch in den Endprodukten niederschlagen: So führt dieses Herstellungsverfahren für die Calciumacetat-Komponente in bezug auf das fertige Produkt zu der zuvor beschriebenen chemischen Instabilität, da die jeweiligen Komponenten in der fertigen Formulierung nicht ausreichend geschützt und in keiner optimalen Struktur bzw. Verteilung eingebunden bzw. verteilt sind und somit Zersetzungsprozesse auftreten können. Zudem weist die Hordentrocknung zur Bereitstellung der Calciumacetat-Komponente eine schlechte Standardisierung bzw. Reproduzierbarkeit auf. Insbesondere ist der Wassergehalt bzw. die Restfeuchte in der mittels Hordentrocknung erhaltenen Calciumacetat-Komponente nur schwer zu regulieren bzw. einzustellen, was im Hinblick auf das Endprodukt ungünstig ist. Der Wassergehalt ist sowohl für die chemische Stabilität als auch für die weitere Verarbeitung des Produktes von großer Bedeutung. Zudem ist es auf Basis der Hordentrocknung oftmals schwierig, definierte Teilchengrößen ohne große Streuung einzustellen. Die mittels Hordentrocknung erhaltenen Komponenten weisen zudem oftmals nur eine ungenügende Fließ- bzw. Rieselfähigkeit auf, was gleichermaßen für einen großtechnischen Herstellungsprozeß ungünstig ist. Zudem sind die mittels Hordentrocknung erhaltenen Komponenten nicht immer ausreichend kompakt, einhergehend mit einer oftmals nur geringen Schüttdichte und einem geringen Wirkstoffgehalt.

Die US 200710128271 A1 offenbart eine pharmazeutische Zusammensetzung, welche ein Calciumacetat und Magnesiumstearat enthaltendes Granulat aufweist, wobei das Granulat durch eine Trockengranulierung hergestellt wird.

Die WO 99/01121 A1 offenbart eine Dosierform zur verzögerten Freisetzung von Sertralin. Bei der Herstellung der Dosierform können calciumacetathaltige Granulate, welche mittels Trockengranulierung zugänglich sind, zum Einsatz kommen. Das calciumacetathaltige Granulat kann weiterhin mit Magnesiumstearat vermischt und zu Tabletten verpreßt werden.

Schließlich offenbart die US-Patentschrift 2,371,862 eine Formulierung für eine Tablette, welche ein Calciumacetatgranulat enthält, wobei das Granulat durch eine Feuchtgranulierung hergestellt wird.

Vor diesem Hintergrund besteht die Aufgabe der vorliegenden Erfindung darin, ein Verfahren zur Herstellung einer eine Kombination von Calciumacetat einerseits und Magnesiumsalz andererseits enthaltenden Zusammensetzung bereitzustellen, wobei die zuvor geschilderten Nachteile des Standes der Technik zumindest weitgehend vermieden oder wenigstens abgeschwächt werden sollen, wobei das Verfahren zu reproduzierbaren Produkten mit verbesserten Eigenschaften führen soll.

Die Anmelderin hat nun in völlig überraschender Weise herausgefunden, daß die zuvor geschilderte Aufgabe dadurch gelöst werden kann, daß erfindungsgemäß ein Verfahren bereitgestellt wird, anhand dessen partikuläres Calciumacetatgranulat zur Herstellung der Zusammensetzung bereitgestellt wird, welches mittels Wirbelschichtgranulation hergestellt wird. Im Rahmen des erfindungsgemäßen Verfahrens wird das mittels Wirbelschichtgranulation hergestellte partikuläre Calciumacetatgranulat mit einer bereitgestellten partikulären Magnesiumsalz-Komponente, gegebenenfalls unter Zugabe weiterer Inhaltsstoffe bzw. Exzipienten, gemischt und nachfolgend zu einer applikationstähigen Formulierung, wie einer Tablette oder dergleichen, verarbeitet. Die auf dieser Basis hergestellte Zusammensetzung bzw. Formulierung eignet sich insbesondere zur prophylaktischen oder therapeutischen Behandlung von mit renalen Dysfunktionen, insbesondere Niereninsuffizienz, einhergehenden Erkrankungen bzw. Störungen, wie Hyperphosphatämie und/oder Hypercalcämie und weist gegenüber Formulierungen des Standes der Technik - wie nachfolgend angeführt - signifikante Vorteile auf.

Bei allen nachstehend genannten relativen bzw. prozentualen gewichtsbezogenen Mengenangaben ist zu beachten, daß diese im Rahmen des erfindungsgemäßen Verfahrens bzw. der erfindungsgemäßen Zusammensetzung derart auszuwählen sind, daß sie sich in der Summe in dem jeweiligen Bezugssystem (z. B. in der erfindungsgemäßen Zusammensetzung oder in dem partikulären Calciumacetatgranulat) unter Einbeziehung der Komponenten bzw. Inhaltsstoffe bzw. Zusatzstoffe bzw. Bestandteile bzw. Exzipienten, insbesondere wie nachfolgend definiert, stets um 100 Gew.-% ergänzen. Dies versteht sich für den Fachmann aber von selbst. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Es versteht sich von selbst, daß Ausgestaltungen, Ausftrungsformen, Vorteile und dergleichen, welche nachfolgend zu Zwecken der Vermeidung von Wiederholungen nur zu einem Erfindungsaspekt angerührt sind, selbstverständlich auch in bezug auf die übrigen Erfindungsaspekte entsprechend gelten.

Gegenstand der vorliegenden Erfindung - gemäß einem **ersten** Aspekt der vorliegenden Erfindung - ist ein Verfahren zur Herstellung einer Zusammensetzung, insbesondere eines Arzneimittels zur prophylaktischen und/oder therapeutischen Behandlung von mit renalen Dysfunktionen, insbesondere Niereninsuffizienz, einhergehenden Erkrankungen und/oder Störungen, wie Hyperphosphatämie und/oder Hypercalcämie, wobei die Zusammensetzung eine Kombination von Calciumacetat einerseits und Magnesiumsalz andererseits aufweist. Das erfindungsgemäße Verfahren zeichnet sich dadurch aus, daß es die folgenden Verfahrensschritte umfaßt:
(a) Bereitstellung von partikulärem Calciumacetatgranulat, wobei das Calciumacetatgranulat mittels Wirbelschichtgranulation hergestellt wird;
(b) Bereitstellung einer partikulären Magnesiumsalz-Komponente;
(c) Herstellung einer Mischung des unter (a) bereitgestellten Calciumacetatgranulates und der unter (b) bereitgestellten Magnesiumsalz-Komponente; und
(d) nachfolgend Verarbeitung der unter (c) hergestellten Mischung zu einer applikationsfähigen, vorzugsweise peroral applizierbaren Formulierung, insbesondere Arzneimittelform.

Die grundlegende Idee der vorliegenden Erfindung ist somit darin zu sehen, daß im Rahmen des erfindungsgemäßen Verfahrens zur Herstellung des partikulären Calciumacetatgranulates eine Wirbelschichtgranulation eingesetzt wird.

Denn die Anmelderin hat in völlig überraschender Weise gefunden, daß der zweckgerichtete Einsatz der Wirbelschichtgranulation zur Bereitstellung von partikulärem Calciumacetatgranulat, welches im Rahmen des erfmdungsgemäßen Verfahrens anschließend mit einer Magnesiumsalz-Komponente vermischt wird, zu entscheidenden Vorteilen führt:

So ist es im Rahmen der vorliegenden Erfindung gelungen, ein Verfahren bereitzustellen, welches in signifikanter Weise die Produkteigenschaften der resultierenden Zusammensetzung verbessert. So führt der zweckgerichtete Einsatz der Wirbelschichtgranulation insbesondere dazu, daß das resultierende Endprodukt in Form der erfindungsgemäßen Zusammensetzung auf Basis von Calciumacetat einerseits und Magnesiumcarbonat andererseits eine signifikant verbesserte Stabilität, insbesondere chemische Stabilität, und damit eine verbesserte Lagerfähigkeit bzw. Haltbarkeit aufweist. Im grundlegenden Unterschied zum Stand der Technik weisen die auf Basis des erfindungsgemäßen Verfahrens hergestellten Zusammensetzungen nach der Erfindung keine Tendenz zur Bildung von Essigsäure und Kohlendioxid auf. Denn die Wirbelschichtgranulation führt in völlig überraschender Weise dazu, daß - ohne sich auf diese Theorie beschränken zu wollen - aufgrund der speziellen Struktur der resultierenden Calciumacetatgranulate einhergehend mit der hohen Kompaktheit im Endprodukt, d. h. in der erfindungsgemäßen Zusammensetzung, eine höhere Stabilität vorliegt, so daß insbesondere Abbaureaktionen von Calciumacetat einerseits und Magnesiumcarbonat andererseits verhindert bzw. deutlich reduziert werden.

Ein weiterer entscheidender Vorteil des erfindungsgemäßen Verfahrens ist darin zu sehen, daß aufgrund des Einsatzes der Wirbelschichtgranulation - im entscheidenden Unterschied zur Hordentrocknung - eine gute Standardisierung der erhaltenen Endprodukte und damit eine ausgezeichnete Reproduzierbarkeit möglich ist. In diesem Zusammenhang hat die Anmelderin völlig überraschend gefunden, daß insbesondere der Wassergehalt bzw. die Restfeuchte des partikulären Calciumacetatgranulates und damit auch die Restfeuchte des Endproduktes gezielt und definiert eingestellt werden können, was gleichermaßen die Verarbeitung bzw. Verpreßbarkeit und die Zerfallseigenschaften der erfindungsgemäßen Zusammensetzung, insbesondere wenn diese in Form von Tabletten oder dergleichen vorliegt, deutlich verbessert. Der Einsatz der Wirbelschichtgranulation führt somit auch zu einer verbesserten physikalischen Stabilität der erhaltenen Endprodukte.

Gleichermaßen können die Teilchengrößen und die Teilchengrößenverteilung des partikulären Calciumacetatgranulates mittels Wirbelschichtgranulation reproduzierbar eingestellt werden, was gleichermaßen die Qualität des Endproduktes verbessert.

Aufgrund der Wirbelschichtgranulation resultiert auch eine verbesserte Fließfähigkeit bzw. Rieselfähigkeit des partikulären Calciumacetatgranulates an sich als auch der in Schritt b) hergestellten Mischung des partikulären Calciumacetatgranulates mit der Magnesiumsalz-Komponente, was insbesondere hinsichtlich eines großtechnischen Verarbeitungsprozesses im industriellen Maßstab von Vorteil ist.

Weiterhin konnte die Anmelderin in völlig überraschender Weise zeigen, daß das mittels Wirbelschichtgranulation hergestellte partikuläre Calciumacetatgranulat bzw. die Calciumacetat-Komponente im Vergleich zur Hordentrocknung über eine erhöhte Dichte bzw. Schüttdichte verfügt, so daß insgesamt kompaktere Granulate mit hohem Wirkstoffgehalt bereitgestellt werden können, was insbesondere hinsichtlich der erfmdungsgemäß bereitgestellten Applikationsform bzw. Formulierung von Vorteil ist.

Weiterhin ist es im Rahmen der vorliegenden Erfindung möglich, aufgrund der Wirbelschichtgranulation im Herstellungsverfahren höhere Durchsätze zu realisieren, worin ein weiterer entscheidender Vorteil gegenüber der mitunter zeit- und kostenintensiven Hordentrocknung zu sehen ist.

Der Einsatz der Wirbelschichtgranulation ist unter produktionstechnischen Gesichtspunkten insbesondere auch insofern vorteilhaft, als die Teilprozesse Granulieren und Trocknen und gegebenenfalls Mischen in einer Vorrichtung vereint werden, so daß produktionstechnisch aufwendige Teilschritte entfallen können. Zudem weist die Wirbelschichtgranulation den Vorteil auf, daß aufgrund der günstigen Wärme- und Stoffübergänge durch Konvektion und Diffusion Vorteile in bezug auf eine Energieeinsparung als auch hinsichtlich der resultierenden Produkteigenschaften resultieren.

Insbesondere weist die mittels des erfindungsgemäßen Verfahrens hergestellte Zusammensetzung nach der Erfindung hinsichtlich der Wirkkomponenten Calciumacetat einerseits und Magnesiumcarbonat andererseits ein verbessertes Freisetzungsprofil auf, was insbesondere auch aufgrund der optimierten Zerfallseigenschaften der erfindungsgemäß bereitgestellten Formulierung zurückzuführen ist. Insbesondere weist die erfindungsgemäße Zusammensetzung eine retardierte bzw. vergleichmäßigte Freisetzung der Wirkkomponenten auf.

Insgesamt konnte die Anmelderin zeigen, daß der zweckgerichtete Einsatz der Wirbelschichtgranulation zur Bereitstellung von partikulärem Calciumacetatgranulat im Hinblick auf die Herstellung einer eine Calciumacetat-Komponente und eine Magnesiumsalz-Komponente enthaltenden Zusammensetzung zu entscheidenden produktionstechnischen Vorteilen führt, welche sich auch im resultierenden Endprodukt, nämlich der erfindungsgemäßen Zusammensetzung, niederschlagen.

Was die erfindungsgemäß zur Bereitstellung von partikulärem Calciumacetatgranulat eingesetzte Wirbelschichtgranulation anbelangt, so ist diese dem Fachmann als solche wohlbekannt und kann in dem Fachmann an sich bekannten Vorrichtungen, wie Wirbelschichttrockner, Wirbelschichtsprühtürmen oder dergleichen, durchgeführt werden. Beispielsweise kann es im Rahmen der vorliegenden Erfindung vorgesehen sein, daß die zu granulierende Calciumacetat-Komponente zunächst mit Wasser und weiteren Zusatzstoffen, wie Klebemitteln oder dergleichen, angeteigt bzw. zu einem Brei angerührt und anschließend in eine Wirbelschichtgranulationsvorrichtung eingebracht wird. Durch eine entsprechende Einstellung des Volumenstromes der Trocknungs- bzw. Prozeßluft, der Lufttemperatur und des Luftdurchsatzes bzw. der Wirbelgeschwindigkeit erfolgt dann in der Wirbelschichtgranulationsvorrichtung eine Granulierung der Calciumacetat-Komponente, wobei insbesondere auf Basis einer zirkulierenden Wirbelschicht Fragmente bzw. Partikel aus dem eingebrachten Brei bzw. Teig herausgelöst und vom Wirbelstrom mitgeführt werden. Die Granulate werden in der Wirbelschicht durch Verdampfen bzw. Verdunsten des Wassers auf einen definierten Restfeuchtegehalt gebildet. Das resultierende Granulat fällt in der Folge aus der Wirbelschicht heraus und insbesondere auf ein Rost am Boden der Vorrichtung, so daß es aus der Vorrichtung abgeführt werden kann. Nachfolgend kann ein Sieben des erhaltenen Granulates erfolgen.

Im Rahmen der vorliegenden Erfindung kann es auch vorgesehen sein, daß die Wirbelschichtgranulation - auch wenn dies weniger bevorzugt ist - in Form einer Wirbelschichtsprühgranulation durchgeführt werden, wobei eine Lösung bzw. Suspension von Calciumacetat in einen Sprühturm eingebracht und in der Folge das Granulat gebildet wird.

Hinsichtlich weiterer Ausführungen zur Wirbelschichtgranulation bzw. zu Granulaten kann verwiesen werden auf Römpp Chemielexikon, 10. Auflage, Band 6, 1999, Stichwörter: "Wirbelschicht" und "Wirbelschichtverfahren", sowie auf Römpp Chemielexikon, 10. Auflage, Band 2, 1997, Stichwort: "Granulate" sowie auf die dort jeweils in bezug genommene Literatur.

Im Rahmen des erfindungsgemäßen Verfahrens kann es vorgesehen sein, daß in Schritt (a) die Wirbelschichtgranulation ausgehend von einer das Calciumacetat enthaltenden Lösung und/oder Suspension durchgeführt wird, insbesondere wobei zur Herstellung der Lösung und/oder Suspension Wasser als Löse- und/oder Suspensionsmittel verwendet wird.

Was die im Rahmen der Wirbelschichtgranulation eingesetzte Lösung bzw. Suspension anbelangt, so sollte der Lösung bzw. Suspension das Calciumacetat in einer Menge von 30 bis 90 Gew.-%, insbesondere 40 bis 80 Gew.-%, vorzugsweise 45 bis 75 Gew.-%, zugegeben werden, bezogen auf die Lösung und/oder Suspension und berechnet als Calciumacetat.

In diesem Zusammenhang kann das Calciumacetat beispielsweise als Calciumacetathydrat eingesetzt werden, wobei der Feuchte- bzw. Wassergehalt, welcher z. B. mittels der dem Fachmann bekannten Karl-Fischer-Titration bestimmt werden kann, bei der Einwage entsprechend zu berücksichtigen ist.

Erfindungsgemäß kann es vorgesehen sein, daß die das Calciumacetat enthaltende Lösung und/oder Suspension das Wasser in einer Menge von 5 bis 30 Gew.-%, insbesondere 8 bis 25 Gew.-%, vorzugsweise 10 bis 20 Gew.-%, bezogen auf die Lösung und/oder Suspension, enthält. Erfindungsgemäß bevorzugt wird somit Wasser als Löse- bzw. Suspensionsmittel eingesetzt.

Weiterhin kann der das Calciumacetat enthaltenden Lösung und/oder Suspension mindestens ein Verarbeitungs- und/oder Granulierhilfsmittel, insbesondere mindestens eine viskositätserhöhende Substanz, vorzugsweise auf Proteinbasis, bevorzugt Gelatine, zugegeben werden, insbesondere in einer Menge von 0,1 bis 3 Gew.-%, insbesondere 0,2 bis 2 Gew.-%, vorzugsweise 0,3 bis 1,5 Gew.-%, bezogen auf die Lösung und/oder Suspension.

In diesem Zusammenhang kann beispielsweise derart verfahren werden, daß zur Herstellung des Calciumacetatgranulates in einem ersten Schritt eine Granulierlösung hergestellt wird, bei welcher das Wasser zunächst erhitzt und anschließend die Gelatine in die heiße Flüssigkeit eingerührt und darin vollständig gelöst wird. Anschließend kann dann insbesondere unter Rühren die Zugabe von Calciumacetat zum Erhalt der das Calciumacetat enthaltenden Lösung und/oder Suspension zugegeben werden. Was die das Calciumacetat enthaltende Lösung bzw. Suspension anbelangt, so sollte diese eine zur Granulierung geeignete Konsistenz aufweisen. So kann die Lösung bzw. Suspension brei- bzw. teigartig eingestellt werden. Sofern eine Wirbelschichtsprühgranulation vorgesehen ist, kann die das Calciumacetat enthaltene Lösung bzw. Suspension auch eine niedrigviskose bzw. flüssige Konsistenz aufweisen.

Erfindungsgemäß kann es zudem vorgesehen sein, daß der das Calciumacetat enthaltenden Lösung und/oder Suspension mindestens ein weiterer Inhaltsstoff, insbesondere aus der Gruppe von Verarbeitungs- und/oder Granulierhilfsmitteln, insbesondere Kohlenhydraten, vorzugsweise (Mais-)Stärke und/oder Saccharose, zugegeben wird. Die diesbezügliche Menge kann im Bereich von 0,5 bis 30 Gew.-%, insbesondere 1 bis 25 Gew.-%, vorzugsweise 3 bis 20 Gew.-%, bezogen auf die Lösung und/oder Suspension, variieren.

Die so erhaltene Suspension bzw. Lösung kann in die Wirbelschichtgranulationsvorrichtung eingebracht und anschließend unter Trocknung granuliert werden.

Dabei kann erfindungsgemäß verfahren werden derart, daß in Schritt (a) die Wirbelschichtgranulation bei einer Temperatur von 35 bis 60 °C, insbesondere 45 bis 50 °C, durchgeführt wird. Die Temperaturangaben beziehen sich dabei insbesondere auf die Temperatur der (Wirbel-)Luft in der Wirbelschichtgranulationsvorrichtung.

Weiterhin kann die in Schritt (a) durchgeführte Wirbelschichtgranulation für eine Dauer von 1 bis 10 Stunden, insbesondere 2 bis 8, bevorzugt 3 bis 6 Stunden, durchgeführt werden.

Wie zuvor angeführt, kann das im Rahmen der Wirbelschichtgranulation erhaltene Calciumacetatgranulat auf eine definierte Restfeuchte bzw. einen definierten Wassergehalt eingestellt werden. In diesem Zusammenhang hat die Anmelderin in überraschender Weise herausgefunden, daß sich hinsichtlich der Stabilität und Verpreßbarkeit der erfindungsgemäßen Zusammensetzung besonders gute Ergebnisse erhalten lassen, wenn das in Schritt (a) erhaltene Calciumacetatgranulat auf eine Restfeuchte von höchstens 3 Gew.-%, insbesondere 0,5 bis 3 Gew.-%, vorzugsweise 1 bis 3 Gew.-%, bevorzugt 1,5 bis 2,5 Gew.-%, bezogen auf das Calciumacetatgranulat, eingestellt wird. Die Bestimmung des Restfeuchtegehaltes kann auf Basis einer Karl-Fischer-Titration oder eines Trocknungsverlustes nach PharmEu durchgeführt werden.

Das in Schritt (a) erhaltene Calciumgranulat kann eine mittlere Teilchengröße, insbesondere bestimmt als D50-Wert, im Bereich von 50 bis 1,500 µm, insbesondere 100 bis 1.000 µm, aufweisen. Die Teilchengröße bzw. Teilchengrößenverteilung kann durch Sieben, Klassieren, Brechen, Sichten oder dergleichen, vorzugsweise durch Sieben, eingestellt werden. Gleichermaßen ist auch ein Zerbrechen bzw. eine Dessaggregierung von Agglomeraten möglich.

Weiterhin kann es erfindungsgemäß vorgesehen sein, daß das in Schritt (a) erhaltene Calciumacetatgranulat auf eine Schüttdichte von 0,45 g/ml bis 0,7 g/ml, insbesondere 0,5 g/ml bis 0,6 g/ml, vorzugsweise mindestens 0,53 g/ml, eingestellt wird.

Wie zuvor angeführt, weist das im Rahmen der vorliegenden Erfindung eingesetzte und mittels Wirbelschichtgranulation erhaltene Calciumacetatgranulat gegenüber den durch Hordentrocknung erhaltenen Granulaten eine erhöhte Dichte bzw. Schüttdichte auf. Im Rahmen der vorliegenden Erfindung handelt es sich bei den Calciumacetatgranulaten um kompakte Granulate, welche einen hohen Wirkstoffgehalt aufweisen. Hierin liegt ein entscheidender Unterschied zur Hordentrocknung, bei welcher im allgemeinen deutlich geringere Schüttdichten erhalten werden.

So kann es im Rahmen der vorliegenden Erfindung vorgesehen sein, daß das in Schritt (a) erhaltene Calciumacetatgranulat das Calciumacetat in einer Menge von 5 bis 99 Gew.-%, insbesondere 40 bis 95 Gew.-%, vorzugsweise 50 bis 90 Gew.-%, bevorzugt 60 bis 85 Gew.-%, bezogen auf das Calciumacetatgranulat und berechnet als Calciumacetat, enthält.

Weiterhin kann das in Schritt (a) erhaltene Calciumacetatgranulat das Calciumacetat in einer Menge von 5 bis 50 Gew.-%, insbesondere 7 bis 40 Gew.-%, vorzugsweise 10 bis 30 Gew.-%, bezogen auf das Calciumacetatgranulat und berechnet als Calcium, enthalten.

Gleichermaßen kommt es erfindungsgemäß in Betracht, daß das in Schritt (a) erhaltene Calciumacetatgranulat außerdem mindestens einen weiteren Inhaltsstoff, insbesondere aus der Gruppe von Verarbeitungs- und/oder Granulierhilfsmitteln, insbesondere Kohlenhydraten, Eiweißen oder deren Mischungen, vorzugsweise (Mais-)Stärke, Saccharose und/oder Gelatine, enthält.

In diesem Zusammenhang kann das in Schritt (a) erhaltene Calciumacetatgranulat Gelatine in einer Menge von 0,15 bis 3,5 Gew.-%, insbesondere 0,25 bis 2,5 Gew.-%, vorzugsweise 0,4 bis 2 Gew.-%, und/oder (Mais-)Stärke in einer Menge von 1 bis 30 Gew.-%, insbesondere 5 bis 25 Gew.-%, vorzugsweise 10 bis 20 Gew.-%, und/oder Saccharose in einer Menge von 1 bis 15 Gew.-%, insbesondere 2 bis 10 Gew.-%, jeweils bezogen auf das Calciumacetatgranulat, enthalten.

Was die in Schritt (b) bereitgestellte Magnesiumsalz-Komponente anbelangt, so kann diese in Form eines Magnesiumsalzgranulates eingesetzt werden. Insbesondere kann das Magnesiumsalzgranulat mittels Granulation, insbesondere Wirbelschichtgranulation, vorzugsweise Wirbelschichtsprühgranulation, hergestellt sein. Grundsätzlich können in bezug auf die Magnesiumsalz-Komponente jedoch alle üblichen Granulationsverfahren eingesetzt werden. Erfindungsgemäß sollte jedoch eine Co-Granulation von Calciumacetat und der Magnesiumsalz-Komponente vermieden werden, insbesondere da dies nachteilig hinsichtlich der chemischen Stabilität der erhaltenen Zusammensetzung ist. Auch aufgrund der erfindungsgemäßen Vorgehensweise, wonach das Calciumacetat mittels Wirbelschichtgranulation und in einem davon getrennten Schritt die Magnesiumsalz-Komponente bereitgestellt werden, können die beiden Komponenten in völlig überraschender Weise zu einer chemisch stabilen Mischung zusammengeführt werden, welche auch in bezug auf die Applikationsform, insbesondere eine Tablette, eine ausgezeichnete Lagerstabilität unter Vermeidung von Essigsäure- und Kohlendioxidbildung aufweist.

Die in Schritt (b) bereitgestellte Magnesiumsalz-Komponente sollte ein physiologisch verträgliches Magnesiumsalz enthalten. Vorzugsweise handelt es sich hierbei um Magnesiumcarbonat. Mit anderen Worten wird als Magnesiumsalz erfindungsgemäß bevorzugt Magnesiumcarbonat verwendet. Hierbei kann es sich auch um basisches Magnesiumcarbonat handeln.

Die in Schritt (b) bereitgestellte Magnesiumsalz-Komponente sollte das Magnesiumsalz, insbesondere Magnesiumcarbonat, in einer Menge von 5 bis 99 Gew.-%, insbesondere 50 bis 98 Gew.-%, vorzugsweise 75 bis 95 Gew.-%, bezogen auf die Magnesiumsalz-Komponente und berechnet als Magnesiumsalz, insbesondere Magnesiumcarbonat, enthalten. Der verbleibende Rest der Magnesiumsalz-Komponente kann beispielsweise durch (Mais-)Stärke gebildet sein bzw. diese enthalten.

Somit kann die in Schritt (b) bereitgestellte Magnesiumsalz-Komponente das Magnesiumsalz, insbesondere Magnesiumcarbonat, in einer Menge von 5 bis 50 Gew.-%, insbesondere 7 bis 40 Gew.-%, vorzugsweise 10 bis 30 Gew.-%, bezogen auf die Magnesiumsalz-Komponente und berechnet als Magnesium, enthalten.

Gleichermaßen ist auch in bezug auf die Magnesiumsalz-Komponente die Zugabe weiterer Inhaltsstoffe, insbesondere wie zuvor in bezug auf das Calciumacetatgranulat definiert, möglich.

Was die Herstellung der Mischung in Schritt (c) anbelangt, so kann dieser Mischung bzw. der resultierenden Zusammensetzung mindestens ein weiterer Inhaltsstoff, insbesondere aus der Gruppe von Füllstoffen, Verarbeitungshilfsmitteln, Gleitmitteln und Sprengmitteln, zugeben werden. Dabei kann die Zugabe in an sich üblicher Weise vor bzw. während des Mischvorganges erfolgen, beispielsweise mittels Einsieben.

In diesem Zusammenhang kann der gegebenenfalls zugegebene Füllstoff ausgewählt werden aus der Gruppe von Lactose, Sorbitol und Saccharose, vorzugsweise Saccharose. Der Füllstoff kann in einer Menge von 0,1 bis 50 Gew.-%, insbesondere 0,2 bis 30 Gew.-%, vorzugsweise 0,5 bis 10 Gew.-%, bezogen auf die Zusammensetzung, zugegeben werden.

In bezug auf das gegebenenfalls zugegebene Gleitmittel kann ein Fettsäuresalz (z. B. ein vorzugsweise pflanzliches Stearat, insbesondere Magnesiumstearat) zugegeben werden. Das Gleitmittel kann in einer Menge von 0,1 bis 50 Gew.-%, insbesondere 0,2 bis 30 Gew.-%, vorzugsweise 0,5 bis 10 Gew.-%, bezogen auf die Zusammensetzung, zugegeben werden.

Was das Sprengmittel, welches synonym auch als Zerfallsbeschleuniger bezeichnet wird, anbelangt, so kann dieses ausgewählt werden aus der Gruppe von Stärke, Cellulose, insbesondere mikrokristalliner Cellulose, und Polyvinylpyrrolidon, vorzugsweise Croscarmellosenatrium. Das Sprengmittel kann in einer Menge von 0,2 bis 20 Gew.-%, insbesondere 0,5 bis 15 Gew.-%, vorzugsweise 1 bis 10 Gew.-%, bezogen auf die Zusammensetzung, zugegeben werden. Der Zerfallsbeschleuniger führt insbesondere zu einer besseren bzw. kontrollierten Auflösung der Formulierung bei deren Applikation, insbesondere im Magen eines Patienten. Der verbesserte Zerfall der Formulierung führt dabei gleichermaßen zu einer Verbesserung bzw. Vergleichmäßigung der Wirkstofffreisetzung. Bei dem Sprengmittel Croscarmellosenatrium handelt es sich insbesondere um ein modifiziertes Cellulosegummi, welches beispielsweise unter der Markenbezeichnung Ac-di-Sol^{®} erhältlich ist.

Weiterhin kann es erfindungsgemäß vorgesehen sein, daß in Schritt (c) der Mischung und/oder der Zusammensetzung mindestens ein weiterer Inhaltsstoff, insbesondere aus der Gruppe von pH-Stellmitteln, pH-Puffersubstanzen, Schmiermitteln, Farbstoffen, Aroma- und Geschmacksstoffen, Geschmackskorrigentien, Stabilisatoren, Konservierungsmitteln, konsistenzsteuemden Mitteln, Verdickungsmitteln, und deren Mischungen, zugegeben wird. Derartige Inhaltsstoffe sind dem Fachmann bekannt, so daß diesbezüglich keine weiteren Ausführungen erforderlich sind.

In Schritt (c) sollte eine zumindest im wesentlichen homogene Vermischung der Komponenten, d. h. der Calciumacetat-Komponente einerseits und der Magnesiumsalz-Komponente andererseits sowie der gegebenenfalls weiteren Inhaltsstoffe, zum Erhalt der erfindungsgemäßen Zusammensetzung durchgeführt werden. Das Vermischen kann in an sich üblicher Weise beispielsweise in einem Mischcontainer durchgeführt werden.

Die im Rahmen des erfindungsgemäßen Verfahrens erhaltene Mischung bzw. Zusammensetzung nach der Erfindung zeichnet sich dadurch aus, daß sie einen hohen Gehalt an Wirkstoffen aufweist:

So kann die in Schritt (c) erhaltene Mischung und/oder die Zusammensetzung das Calciumacetat in einer Menge von 5 bis 30 Gew.-%, insbesondere 6 bis 25 Gew.-%, vorzugsweise 8 bis 20 Gew.-%, bezogen auf die Mischung und/oder Zusammensetzung und berechnet als Calcium, enthalten.

In bezug auf das Magnesium kann die in Schritt (c) erhaltene Mischung und/oder die Zusammensetzung das Magnesiumsalz, insbesondere Magnesiumcarbonat, in einer Menge von 1 bis 20 Gew.-%, insbesondere 2 bis 15 Gew.-%, vorzugsweise 3 bis 10 Gew.-%, bevorzugt 4 bis 8 Gew.-%, bezogen auf Mischung und/oder Zusammensetzung und berechnet als Magnesium, enthalten.

Im Rahmen der vorliegenden Erfindung hat es sich insbesondere hinsichtlich der Stabilität der mittels des erfindungsgemäßen Verfahrens hergestellten Zusammensetzung nach der Erfindung sowie hinsichtlich ihrer Wirksamkeit zur Behandlung der oben genannten Erkrankungen bzw. Störungen als besonders vorteilhaft herausgestellt, wenn die in Schritt (c) erhaltene Mischung und/oder die Zusammensetzung ein Calciumacetat/Magnesiumsalz-Verhältnis, insbesondere ein Calciumacetat/Magnesiumcarbonat-Verhältnis, berechnet als Calcium einerseits und Magnesium andererseits, im Bereich von 10 : 1 bis 1 : 5, insbesondere 5 : 1 bis 1 : 2, vorzugsweise 4 : 1 bis 1 : 1, bevorzugt 2,5 : 1 bis 1,2 : 1, aufweist.

Erfindungsgemäß kann es gleichermaßen vorgesehen sein, daß die im Rahmen des erfindungsgemäßen Verfahrens erhältliche Zusammensetzung nach der Erfindung mindestens einen Exzipienten bzw. Träger aufweist. Dieser kann Bestandteil des in Schritt (a) hergestellten Calciumacetatgranulates und/oder der in Schritt (b) bereitgestellten Magnesiumsalz-Komponente sein. Gleichermaßen kann der Exzipient bzw. Träger auch separat zugesetzt werden, d. h. der Zusammensetzung als solcher zugegeben werden.

Zur Herstellung der applikationsfähigen Formulierung kann es in Schritt (d) des erfindungsgemäßen Verfahrens vorgesehen sein, daß eine Verarbeitung der Mischung und/oder der Zusammensetzung zu Kapseln, Tabletten, insbesondere Filmtabletten oder Dragees, applikationsfähigen Granulaten oder dergleichen erfolgt, insbesondere wobei die Verarbeitung mittels Verpressen erfolgen kann. Beispielsweise kann die Herstellung von Tabletten mit Hilfe einer Rundlaufpresse erfolgen.

Was die applikationsfähige Formulierung weiterhin anbelangt, so kann die in Schritt (d) erhaltene Formulierung bzw. Arzneimittelform eine Masse von 100 bis 2.000 mg, insbesondere 250 bis 1.750 mg, vorzugsweise 500 bis 1.500, bevorzugt 700 bis 1.200 mg, aufweisen. In besonders bevorzugter Art und Weise kann die applikationsfähige Formulierung eine Masse von etwa 900 mg aufweisen. Die vorgenannten Werte beziehen sich somit auf die fertige Applikations- bzw. Dosiereinheit, beispielsweise in Form einer Tablette.

Die in Schritt (d) erhaltene applikationsfähige Formulierung, insbesondere die fertige Applikationseinheit, beispielsweise in Form einer Tablette, kann das Calciumacetat in einer Menge von 50 bis 200 mg, insbesondere 75 bis 150 mg, vorzugsweise 90 bis 120 mg, bevorzugt etwa 110 mg, berechnet als Calcium, aufweisen.

In diesem Zusammenhang kann die in Schritt (d) erhaltene applikationsfähige Formulierung, insbesondere die fertige Applikations- bzw. Dosiereinheit, beispielsweise in Form einer Tablette, das Magnesiumsalz, insbesondere das Magnesiumcarbonat, in einer Menge von 20 bis 100 mg, insbesondere 30 bis 80 mg, vorzugsweise 40 bis 70 mg, bevorzugt etwa 60 mg, berechnet als Magnesium, aufweisen.

Im Rahmen der vorliegenden Erfindung kann es weiterhin vorgesehen sein, daß die in Schritt (d) erhaltene Formulierung mit einer vorzugsweise magensaftlöslichen Beschichtung versehen wird. Dies kann in dem Fachmann an sich bekannter Art und Weise mittels Beschichtung bzw. Lackieren erfolgen. In diesem Zusammenhang kann beispielsweise eine ein Rizinusöl und Hydroxypropylmethylcellulose enthaltende Beschichtung aufgebracht werden.

Weiterer Gegenstand der vorliegenden Erfindung - gemäß einem **zweiten** Aspekt der vorliegenden Erfindung - ist eine Zusammensetzung, insbesondere ein Arzneimittel zur prophylaktischen und/oder therapeutischen Behandlung von mit renalen Dysfunktionen, insbesondere Niereninsuffizienz, einhergehenden Erkrankungen und/oder Störungen, wie Hyperphosphatämie und/oder Hypercalcämie, wobei die Zusammensetzung eine Kombination von Calciumacetat einerseits und Magnesiumsalz andererseits aufweist. Die erfindungsgemäße Zusammensetzung zeichnet sich dadurch aus, daß die Zusammensetzung erhältlich ist durch das zuvor beschriebene bzw. definierte Verfahren.

Weiterhin betrifft die vorliegende Erfindung - gemäß einem **dritten** Aspekt der vorliegenden Erfindung - eine Zusammensetzung, insbesondere ein Arzneimittel zur prophylaktischen und/oder therapeutischen Behandlung von mit renalen Dysfunktionen, insbesondere Niereninsuffizienz, einhergehenden Erkrankungen und/oder Störungen, wie Hyperphosphatämie und/oder Hypercalcämie, wobei die Zusammensetzung - in jeweils wirksamen, insbesondere pharmazeutisch wirksamen Mengen und zusammen mit mindestens einem Exzipienten - eine Kombination von Calciumacetat einerseits und Magnesiumsalz andererseits aufweist. Die erfindungsgemäße Zusammensetzung gemäß diesem Aspekt zeichnet sich dadurch aus, daß das Calciumacetat in Form eines mittels Wirbelschichtgranulation hergestellten Calciumacetatgranulates eingesetzt ist.

Die erfindungsgemäße Zusammensetzung gemäß dem zweiten und dritten Aspekt der vorliegenden Erfindung weist - wie zuvor zu dem ersten Aspekt der vorliegenden Erfindung angeführt - eine hohe chemische Stabilität auf, was zu hervorragenden Lagereigenschaften führt. Dabei ist es im Rahmen der vorliegenden Erfindung gelungen, aufgrund des Einsatzes von Calciumacetat in Form eines mittels Wirbelschichtgranulation hergestellten Calciumacetatgranulates die Wirkstoffkomponenten Calciumacetat einerseits und Magnesiumcarbonat andererseits in optimaler Weise aufeinander abzustimmen, was insgesamt auch zu einer erhöhten Wirkeffizienz der erfindungsgemäßen Zusammensetzung im Hinblick auf die oben genannten Erkrankungen bzw. Störungen, insbesondere im Hinblick auf die Behandlung von Hyperphosphatämie und damit einhergehender Hypercalcämie, führt.

Die Zusammensetzung nach der Erfindung kann das Calciumacetat in einer Menge von 5 bis 30 Gew.-%, insbesondere 6 bis 25 Gew.-%, vorzugsweise 8 bis 20 Gew.-%, bezogen auf die Zusammensetzung und berechnet als Calcium, enthalten.

Weiterhin kann es vorgesehen sein, daß die erfindungsgemäße Zusammensetzung das Calciumacetat pro Applikationseinheit, insbesondere in Form einer Tablette, bzw. pro Tablette, in einer Menge von 50 bis 200 mg, insbesondere 75 bis 150 mg, vorzugsweise 90 bis 120 mg, bevorzugt etwa 110 mg, berechnet als Calcium, aufweist.

Das Magnesiumsalz sollte ein physiologisch verträgliches Magnesiumsalz sein, wobei der Einsatz von Magnesiumcarbonat besonders vorteilhaft ist.

Die Zusammensetzung nach der Erfindung kann das Magnesiumsalz, insbesondere Magnesiumcarbonat, in einer Menge von 1 bis 20 Gew.-%, insbesondere 2 bis 15 Gew.-%, vorzugsweise 3 bis 10 Gew.-%, bevorzugt 4 bis 8 Gew.-%, bezogen auf das Magnesiumsalz, insbesondere Magnesiumcarbonat, und berechnet als Magnesium, enthalten.

Diesbezüglich kann die Zusammensetzung pro Applikationseinheit insbesondere in Form einer Tablette, bzw. pro Tablette, das Magnesiumsalz, insbesondere das Magnesiumcarbonat, in einer Menge von 20 bis 100 mg, insbesondere 30 bis 80 mg, vorzugsweise 40 bis 70 mg, bevorzugt etwa 60 mg, berechnet als Magnesium, aufweisen.

Gleichermaßen ist es besonders vorteilhaft, wenn die erfindungsgemäße Zusammensetzung ein Calciumacetat/Magnesiumsalz-Verhältnis, insbesondere ein Calciumacetat/Magnesiumcarbonat-Verhältnis, berechnet als Calcium einerseits und Magnesium andererseits, im Bereich von 10 : 1 bis 1 : 5, insbesondere 5 : 1 bis 1 : 2, vorzugsweise 4: 1 bis 1 : 1, bevorzugt 2,5 : 1 bis 1,2 : 1, aufweist.

Die erfindungsgemäße Zusammensetzung kann mindestens einen weiteren Inhaltsstoff, insbesondere aus der Gruppe von Füllstoffen, Verarbeitungshilfsmitteln, Gleitmitteln und Sprengmitteln, aufweisen.

Beispielsweise kann der Füllstoff ausgewählt sein aus der Gruppe von Lactose, Sorbitol und Saccharose, vorzugsweise Saccharose. Der Füllstoff kann in der Zusammensetzung in einer Menge von 0,1 bis 50 Gew.-%, insbesondere 0,2 bis 30 Gew.-%, vorzugsweise 0,5 bis 10 Gew.-%, bezogen auf die Zusammensetzung, vorliegen.

Das Gleitmittel kann beispielsweise als Fettsäuresalz (z. B. als vorzugsweise pflanzliches Stearat, insbesondere Magnesiumstearat) zugegeben sein. Das Gleitmittel kann in der Zusammensetzung in einer Menge von 0,1 bis 50 Gew.-%, insbesondere 0,2 bis 30 Gew.-%, vorzugsweise 0,5 bis 10 Gew.-%, bezogen auf die Zusammensetzung, vorliegen.

Was das gegebenenfalls vorhandene Sprengmittel anbelangt, so kann dieses ausgewählt sein aus der Gruppe von Stärke, Cellulose, insbesondere mikrokristalliner Cellulose, und Polyvinylpyrrolidon, vorzugsweise Croscarmellosenatrium. Das Sprengmittel kann in der Zusammensetzung in einer Menge von 0,2 bis 20 Gew.-%, insbesondere 0,5 bis 15 Gew.-%, vorzugsweise 1 bis 10 Gew.-%, bezogen auf die Zusammensetzung, vorliegen.

Die Zusammensetzung kann zudem mindestens einen weiteren Inhaltsstoff, insbesondere aus der Gruppe von pH-Stellmitteln, pH-Puffersubstanzen, Schmiermitteln, Farbstoffen, Aroma- und Geschmacksstoffen, Geschmackskorrigentien, Stabilisatoren, Konservierungsmitteln, konsistenzsteuemde Mittel, Verdickungsmittel, und deren Mischungen, aufweisen. Zudem kann die Zusammensetzung übliche Exzipienten bzw. Träger enthalten.

Erfindungsgemäß kann es vorgesehen sein, daß die Zusammensetzung verpreßt ist. Auf diese Weise kann insbesondere eine applikationsfertige Formulierung bzw. Arzneimittelform, wie nachfolgend beschrieben, erhalten werden.

So kann die erfindungsgemäße Zusammensetzung in Form einer applikationsfähigen, vorzugsweise peroral applizierbaren Formulierung, insbesondere Arzneimittelform, vorliegen, insbesondere wobei die Formulierung eine Masse von 100 bis 2.000 mg, insbesondere 250 bis 1.750 mg, vorzugsweise 500 bis 1.500, bevorzugt 700 bis 1.200 mg, aufweisen kann und/oder insbesondere wobei die Formulierung mit einer vorzugsweise magensaftlöslichen Beschichtung versehen sein kann.

Die Zusammensetzung kann in Form von applikationsfähigen Granulaten, Kapseln, Tabletten, insbesondere Filmtabletten oder Dragees oder dergleichen vorliegen.

Für weitere diesbezügliche Angaben zu dem zweiten und dritten Aspekt der vorliegenden Erfindung kann auf die Ausführungen zu den weiteren Aspekten der vorliegenden Erfindung verwiesen werden, welche für die erfindungsgemäße Zusammensetzung entsprechend gelten.

Zudem betrifft die vorliegende Erfindung - gemäß einem **vierten** Aspekt der vorliegenden Erfindung - die Zusammensetzung, insbesondere das Arzneimittel, wie zuvor definiert, zur Anwendung in der prophylaktischen und/oder therapeutischen Behandlung von mit renalen Dysfunktionen, insbesondere Niereninsuffizienz, einhergehenden Erkrankungen und/oder Störungen, insbesondere Hyperphosphatämie und/oder Hypercalcämie.

Weiterhin betrifft die vorliegende Erfindung - gemäß einem **fünften** Aspekt der vorliegenden Erfindung - die Verwendung einer Kombination von Calciumacetat einerseits und Magnesiumsalz andererseits zur Herstellung einer Zusammensetzung, insbesondere eines Arzneimittels zur prophylaktischen und/oder therapeutischen Behandlung von mit renalen Dysfunktionen, insbesondere Niereninsuffizienz, einhergehenden Erkrankungen und/oder Störungen, insbesondere Hyperphosphatämie und/oder Hypercalcämie. Die erfindungsgemäße Verwendung der vorgenannten Kombination ist **dadurch gekennzeichnet, daß** das Calciumacetat in Form eines mittels Wirbelschichtgranulation hergestellten Calciumacetatgranulates eingesetzt wird.

Die erfindungsgemäße Verwendung der Kombination von Calciumacetat einerseits und Magnesiumsalz andererseits kann insbesondere zur Behandlung von Dialysepatienten und/oder Diabetespatienten realisiert werden. Aufgrund der erfindungsgemäßen Konzeption wird eine besonders gute Wirksamkeit in bezug auf eine Verminderung von Hyperphosphatämie bzw. Hypercalcämie erreicht.

Für weitere diesbezügliche Angaben kann verwiesen werden auf die Ausführungen zu den weiteren Aspekten der vorliegenden Erfindung, welche für die erfindungsgemäße Verwendung gemäß dem vierten Aspekt und dem fünften Aspekt der vorliegenden Erfindung entsprechend gelten.

Die vorliegende Erfindung betrifft weiterhin - gemäß einem **sechsten** Aspekt der vorliegenden Erfindung ein Verfahren zur Herstellung von partikulärem Calciumacetat, wobei das erfindungsgemäße Verfahren dadurch gekennzeichnet ist, daß das Calciumacetat mittels Wirbelschichtgranulation in Form von Calciumacetatgranulat hergestellt wird.

Gemäß einem **siebten** Aspekt der vorliegenden Erfindung betrifft die vorliegende Erfindung partikuläres Calciumacetat, welches in Form von Calciumacetatgranulat mittels Wirbelschichtgranulation erhältlich ist.

Das partikuläre Calciumacetat in Form von Calciumacetatgranulat nach der Erfindung kann außerdem mindestens einen weiteren Inhaltsstoff, insbesondere wie zuvor definiert, enthalten.

In diesem Zusammenhang kann der Inhaltsstoff ausgewählt sein aus der Gruppe von Verarbeitungs- und/oder Granulierhilfsmitteln, insbesondere Kohlenhydraten, Eiweißen oder deren Mischungen, vorzugsweise (Mais-)-Stärke, Saccharose und/oder Gelatine. Zudem können dem Calciumacetatgranulat Exzipienten bzw. Träger zugegeben sein.

Schließlich betrifft die vorliegende Erfindung - gemäß einem **achten** Aspekt der vorliegenden Erfindung - ein Arzneimittel, insbesondere zur prophylaktischen und/oder therapeutischen Behandlung von mit renalen Dysfunktionen, insbesondere Niereninsuffizienz, einhergehenden Erkrankungen oder Störungen, wie Hyperphosphatämie und/oder Hypercalcämie. Das erfindungsgemäße Arzneimittel enthält ein wie zuvor definiertes partikuläres Calciumacetat zusammen mit mindestens einem Exzipienten.

Für weitere diesbezügliche Angaben kann auf die obigen Ausführungen zu den weiteren Aspekten der vorliegenden Erfindung verwiesen werden, welche für den siebten und achten Aspekt der vorliegenden Erfindung entsprechend gelten.

### Ausführungsbeispiele:

### 1. Zusammensetzung A (erfindungsgemäß):

Eine eine Kombination von Calciumacetat und Magnesiumsalz enthaltende Zusammensetzung nach der Erfindung wurde auf Basis des erfindungsgemäßen Verfahrens unter Einsatz der Wirbelschichtgranulation zur Bereitstellung des Calciumacetatgranulates hergestellt. Die nachfolgende Tabelle zeigt die Komponenten bzw. Inhaltsstoffe der erfindungsgemäßen Zusammensetzung:

| **Inhaltsstoffe** | **Menge in mg pro Tablette** |
|---|---|
| *Wirkstoffgranulat I:* | |
| Calciumacetathydrat* | 457,89 |
| Maisstärke | 86,01 |
| Saccharose (Puderzucker) | 39,99 |
| | |
| Gelatine | 5 |
| | |

| *Wirkstoff II:* | |
|---|---|
| Magnesiumcarbonat (Destab Magnesium (90 %)** | 261,1 |
| | |

| *Hilfsstoffe:* | |
|---|---|
| Croscarmellosenatrium (Ac-di-Sol^{®}) | 30 |
| Saccharose (Puderzucker) | 10 |
| | |

| *Gleitmittel:* | |
|---|---|
| Magnesiumstearat (pflanzlich) | 10 |
| | |
| Gewicht/Tablette: | ca. 900 |

| | |
|---|---|
| *) Eine Feuchtigkeit von 5 % Wasser wurde bei der Einwage an Calciumacetat berücksichtigt. **) Es wurde ein Gehalt von 90 % Magnesiumcarbonat berücksichtigt. | |

Der Ausgleich für die Korrektur gemäß *) und gemäß **) erfolgte durch Maisstärke auf ein Preßgewicht von ca. 900 mg.

Zur Herstellung der erfindungsgemäßen Zusammensetzung wurde in einem ersten Schritt eine Granulierlösung zubereitet, wobei die Gelatine in erwärmtes Wasser aufgelöst wurde. Diesbezüglich wurde 1 Gewichtsteil Gelatine auf 24 Gewichtsteile Wasser gegeben.

Das Calciumacetathydrat, die Maisstärke und die Saccharose wurden in einen Pharmamischer homogen gemischt, und anschließend wurde das homogene Gemisch zu der Granulierlösung hinzugegeben und darin unter Rühren eingearbeitet bzw. aufgelöst. Es resultierte ein Brei mit teigartiger Konsistenz, welcher nachfolgend in eine Wirbelschichtgranuliervorrichtung gegeben wurde. Die Granulierzeit betrug 10 min, wobei die Wirkstoffgranulate in der Wirbelschicht bis zu einer Restfeuchte von etwa 2 Gew.-% getrocknet wurden.

Nach dem Trocknen wurden die das Calciumacetat enthaltenden Wirkstoffgranulate sowie das bereitgestellte Magnesiumcarbonat und das Croscarmellosenatrium eingesiebt. Anschließend wurde für etwa 15 min in einem Bohlecontainer gemischt.

Vor dem Verpressen der Zusammensetzung wurden die Saccharose und das Magnesiumstearat eingesiebt und nochmals für etwa 5 min gemischt.

Die Tablettierung erfolgte durch Verpressen der Zusammensetzung, wobei die resultierenden Tabletten ein Preßgewicht von etwa 900 mg bei einer Form von etwa 7,5 x 21,5 mm oblong mit Bruchrille aufwiesen.

Nach erfolgter Tablettierung wurden die Preßlinge mit einer Beschichtung bzw. einem Coating mittels Lackieren ausgerüstet. Diesbezüglich wurde eine Beschichtung gewählt, welche auf 63 Gewichtsteile Wasser 0,9 Gewichtsteile Rizinusöl und 5,1 Gewichtsteile Hydroxypropylmethylcellulose aufwies. Zur Herstellung der Beschichtungslösung wurde zunächst das gereinigte Wasser auf etwa 90 °C (80 °C bis 100 °C) erhitzt und das Rizinusöl darin gelöst bzw. emulgiert. Die Lösung wurde auf eine Temperatur unterhalb von etwa 50 °C abgekühlt. Anschließend wurde die Hydroxypropylmethylcellulose in der abgekühlten Mischung gelöst.

Die so bereitgestellte homogene Lösung wurde für die Beschichtung bzw. das Befilmen der Preßlinge in eine Beschichtungsvorrichtung überführt.

Bei der Befilmung bzw. Beschichtung wurde die Zu- und Abluft so eingestellt, daß die Produkttemperatur etwa 35 °C bis 40 °C betrug. Zur Optimierung des Prozesses wurde die Produkttemperatur bei Bedarf innerhalb von 30 °C bis 50 °C angepaßt.

Zur Beginn der Befilmung bzw. Beschichtung betrug die Umdrehungsgeschwindigkeit 2 bis 4 U/Min. Diese wurde nach kurzer Zeit innerhalb von 3 bis 7 U/Min optimal eingestellt. Zur Prozeßoptinaierung wurde bei Bedarf die Umdrehungsgeschwindigkeit im Bereich von 2 bis 10 U/Min eingestellt.

Es resultierten beschichtete Preßlinge in Form von Filmtabletten, wobei die Beschichtung ein Gewicht von etwa 20 mg pro Filmtablette aufwies. Es resultierten somit Filmtabletten mit einem Gesamtgewicht von etwa 920 mg.

Die auf diese Weise erhaltenen Filmtabletten mit der erfindungsgemäßen Zusammensetzung können in an sich üblicher Weise konfektioniert werden.

### 2. Vergleichszusammensetzung B (nichterfindungsgemäß):

In analoger Weise wurde eine nichterfindungsgemäße Zusammensetzung hergestellt, wobei die Calciumacetat-Komponente in herkömmlicher Weise mittels Hordentrocknung hergestellt worden ist.

### 3. Vergleich der Zusammensetzungen sowie Anwendungstests:

a) Während die Calciumacetatgranulate für die erfindungsgemäße Zusammensetzung A eine Schüttdichte von 0,6 g/ml aufwiesen, besaßen die entsprechenden Granulate der nichterfindungsgemäßen Zusammensetzung B auf Basis einer Hordentrocknung eine Schüttdichte von lediglich 0,5 g/ml. Die für die erfindungsgemäße Zusammensetzung A verwendeten Calciumacetatgranulate waren damit deutlich kompakter als diejenigen für die nichterfindungsgemäße Zusammensetzung B.
b) Auf Basis eines standardisierten Alterungstests wurde die Langzeitstabilität bzw. die Lagerstabilität der Zusammensetzungen A und B überprüft. Diesbezüglich wurden jeweils 10 Filmtabletten der erfindungsgemäßen Zusammensetzung A und der nichterfindungsgemäßen Zusammensetzung B unter beschleunigten Alterungsbedingungen bei 65 % relativer Luftfeuchtigkeit und einer Umgebungstemperatur von 80 °C über einen Zeitraum von zwei Monaten gelagert. Nach einem Monat und nach zwei Monaten wurde der Calciumacetatgehalt in den jeweiligen Zusammensetzungen im Vergleich zum Calciumacetatgehalt zu Versuchsbeginn (100 % bei t = 0) bestimmt. Während bei der erfindungsgemäßen Zusammensetzung A der Gehalt an Calciumacetat nach einem Monat 99,5 % und nach zwei Monaten 98,7 % betrug, wurde in bezug auf die nichterfindungsgemäße Zusammensetzung ein deutlicher Abbau in bezug auf Calciumacetat beobachtet: So wurde nach einem Monat ein Calciumacetatgehalt von 90,1 % und nach zwei Monaten von lediglich 82,3 % ermittelt. Zudem war in bezug auf die nichterfindungsgemäße Zusammensetzung B nach Beendigung des Versuches ein deutlicher Essiggeruch wahrzunehmen. Die erfindungsgemäße Zusammensetzung A, bei welcher durch Wirbelschichtgranulation erhaltenes Calciumacetatgranulat eingesetzt wurde, weist somit gegenüber der nichterfindungsgemäßen Zusammensetzung B, bei welcher die Calciumacetat-Komponente auf Basis einer Hordentrocknung eingesetzt wurde, eine deutlich verbesserte chemische Stabilität und damit eine verlängerte Haltbarkeit bzw. Lagerfähigkeit auf.
c) Schließlich wurde in bezug auf die erfindungsgemäße Zusammensetzung A und die nichterfindungsgemäße Zusammensetzung B ein Freisetzungsversuch zur Ermittlung der Freisetzung von Calciumacetat aus der jeweiligen Zusammensetzung durchgeführt. Diesbezüglich wurde jeweils eine Filmtablette auf Basis der erfindungsgemäßen Zusammensetzung A und eine Filmtablette auf Basis der nichterfindungsgemäßen Zusammensetzung B in 0,1 N Salzsäure unter Rühren aufgelöst und die Freisetzung von Calciumacetat jeweils photometrisch bestimmt. Die Versuchsdurchführung wurde jeweils fünfmal wiederholt. Die erfindungsgemäße Zusammensetzung A wies gegenüber der nichterfindungsgemäßen Zusammensetzung B eine kontrollierte und vergleichmäßigte Freisetzung der Wirkstoffkomponente auf, wobei der Freisetzungsverlauf nahezu linear war. Zudem war der Zeitraum der Freisetzung gegenüber der nichterfindungsgemäßen Zusammensetzung B um etwa 50 % verlängert. Die erfindungsgemäße Zusammensetzung weist somit gegenüber dem Stand der Technik auch ein deutlich verbessertes Freisetzungsverhalten der Wirkstoffkomponenten auf.

Die zuvor beschriebenen Untersuchungen und Vergleiche belegen insgesamt die hervorragenden Eigenschaften der erfindungsgemäßen Zusammensetzung gegenüber dem Stand der Technik, wobei die erfindungsgemäße Zusammensetzung sowohl über eine höhere Lagerstabilität bzw. Alterungsbeständigkeit als auch über ein verbessertes Freisetzungsprofil der Wirkkomponenten verfügt.

## Patentansprüche

1. Verfahren zur Herstellung einer Zusammensetzung, insbesondere eines Arzneimittels zur prophylaktischen und/oder therapeutischen Behandlung von mit renalen Dysfunktionen, insbesondere Niereninsuffizienz, einhergehenden Erkrankungen und/oder Störungen, wie Hyperphosphatämie und/oder Hypercalcämie, wobei die Zusammensetzung eine Kombination von Calciumacetat einerseits und Magnesiumsalz andererseits aufweist,
**dadurch gekennzeichnet,**
**daß** das Verfahren die folgenden Verfahrensschritte umfaßt:
(a) Bereitstellung von partikulärem Calciumacetatgranulat, wobei das Calciumacetatgranulat mittels Wirbelschichtgranulation hergestellt wird;
(b) Bereitstellung einer partikulären Magnesiumsalz-Komponente;
(c) Herstellung einer Mischung des unter (a) bereitgestellten Calciumacetatgranulates und der unter (b) bereitgestellten Magnesiumsalz-Komponente; und
(d) nachfolgend Verarbeitung der unter (c) hergestellten Mischung zu einer applikationstähigen, vorzugsweise peroral applizierbaren Formulierung, insbesondere Arzneimittelform.

2. Verfahren nach Anspruch 1, **dadurch gekennzeichnet, daß** in Schritt (a) die Wirbelschichtgranulation ausgehend von einer das Calciumacetat enthaltenden Lösung und/oder Suspension durchgeführt wird, insbesondere wobei zur Herstellung der Lösung und/oder Suspension Wasser als Löse- und/oder Suspensionsmittel verwendet wird, insbesondere wobei der Lösung und/oder Suspension das Calciumacetat in einer Menge von 30 bis 90 Gew.-%, insbesondere 40 bis 80 Gew.-%, vorzugsweise 45 bis 75 Gew.-%, zugegeben wird, bezogen auf die Lösung und/oder Suspension und berechnet als Calciumacetat, und/oder insbesondere wobei die das Calciumacetat enthaltende Lösung und/oder Suspension das Wasser in einer Menge von 5 bis 30 Gew.-%, insbesondere 8 bis 25 Gew.-%, vorzugsweise 10 bis 20 Gew.-%, bezogen auf die Lösung und/oder Suspension, enthält,

3. Verfahren nach Anspruch 2, **dadurch gekennzeichnet, daß** der das Calciumacetat enthaltenden Lösung und/oder Suspension mindestens ein Verarbeitungs- und/oder Granulierhilfsmittel, insbesondere mindestens eine viskositätserhöhende Substanz, vorzugsweise auf Proteinbasis, bevorzugt Gelatine, zugegeben wird, insbesondere in einer Menge von 0,1 bis 3 Gew.-%, insbesondere 0,2 bis 2 Gew.-%, vorzugsweise 0,3 bis 1,5 Gew.-%, bezogen auf die Lösung und/oder Suspension, insbesondere wobei der das Calciumacetat enthaltenden Lösung und/oder Suspension mindestens ein weiterer Inhaltsstoff, insbesondere aus der Gruppe von Verarbeitungs- und/oder Granulierhilfsmitteln, insbesondere Kohlenhydraten, vorzugsweise (Mais-)Stärke und/oder Saccharose, zugegeben wird, insbesondere in einer Menge von 0,5 bis 30 Gew.-%, insbesondere 1 bis 25 Gew.-%, vorzugsweise 3 bis 20 Gew.-%, bezogen auf die Lösung und/oder Suspension.

4. Zusammensetzung, insbesondere Arzneimittel zur prophylaktischen und/oder therapeutischen Behandlung von mit renalen Dysfunktionen, insbesondere Niereninsufnzienz, einhergehenden Erkrankungen und/oder Störungen, wie Hyperphosphatämie und/oder Hypercalcämie, wobei die Zusammensetzung eine Kombination von Calciumacetat einerseits und Magnesiumsalz andererseits aufweist,
**dadurch gekennzeichnet,**
**daß** die Zusammensetzung erhältlich ist durch ein Verfahren nach einem der vorangehenden Ansprüche.

5. Zusammensetzung, insbesondere Arzneimittel zur prophylaktischen und/oder therapeutischen Behandlung von mit renalen Dysfunktionen, insbesondere Niereninsuffizienz, einhergehenden Erkrankungen und/oder Störungen, wie Hyperphosphatämie und/oder Hypercalcämie, wobei die Zusammensetzung - in jeweils wirksamen, insbesondere pharmazeutisch wirksamen Mengen und zusammen mit mindestens einem Exzipienten - eine Kombination von Calciumacetat einerseits und Magnesiumsalz andererseits aufweist,
**dadurch gekennzeichnet,**
**daß** das Calciumacetat in Form eines mittels Wirbelschichtgranulation hergestellten Calciumacetatgranulates eingesetzt ist.

6. Zusammensetzung nach Anspruch 4 oder 5, **dadurch gekennzeichnet, daß** die Zusammensetzung das Calciumacetat in einer Menge von 5 bis 30 Gew.-%, insbesondere 6 bis 25 Gew.-%, vorzugsweise 8 bis 20 Gew.-%, bezogen auf die Zusammensetzung und berechnet als Calcium, enthält und/oder daß die Zusammensetzung das Calciumacetat pro Applikationseinheit, insbesondere pro Tablette, in einer Menge von 50 bis 200 mg, insbesondere 75 bis 150 mg, vorzugsweise 90 bis 120 mg, bevorzugt etwa 110 mg, berechnet als Calcium, aufweist.

7. Zusammensetzung nach einem der Ansprüche 4 bis 7, **dadurch gekennzeichnet, daß** das Magnesiumsalz ein physiologisch erträgliches Magnesiumsalz, vorzugsweise Magnesiumcarbonat, ist und/oder daß die Zusammensetzung das Magnesiumsalz, insbesondere Magnesiumcarbonat, in einer Menge von 1 bis 20 Gew.-%, insbesondere 2 bis 15 Gew.-%, vorzugsweise 3 bis 10 Gew.-%, bevorzugt 4 bis 8 Gew.-%, bezogen auf die Zusammensetzung und berechnet als Magnesium, enthält und/oder daß die Zusammensetzung das Magnesiumsalz, insbesondere Magnesiumcarbonat, pro Applikationseinheit, insbesondere pro Tablette, in einer Menge von 20 bis 100 mg, insbesondere 30 bis 80 mg, vorzugsweise 40 bis 70 mg, bevorzugt etwa 60 mg, berechnet als Magnesium, aufweist.

8. Zusammensetzung nach einem der Ansprüche 4 bis 7, **dadurch gekennzeichnet, daß** die Zusammensetzung ein Calciumacetat/Magnesiumsalz-Verhältnis, insbesondere ein Calciumacetat/Magnesiumcarbonat-Verhaltnis, berechnet als Calcium einerseits und Magnesium andererseits, im Bereich von 10 : 1 bis 1 : 5, insbesondere 5 : 1 bis 1 : 2, vorzugsweise 4 : 1 bis 1 : 1, bevorzugt 2,5 : 1 bis 1,2 : 1, ausweist.

9. Zusammensetzung nach einem der Ansprüche 4 bis 8, **dadurch gekennzeichnet, daß** die Zusammensetzung mindestens einen weiteren Inhaltsstoff, insbesondere aus der Gruppe von Füllstoffen, Verarbeitungshilfsmittels, Gleitmitteln und Sprengmitteln, aufweist, insbesondere wobei der Füllstoff ausgewählt ist aus der Gruppe von Lactose, Sorbitol und Saccharose, vorzugsweise Saccharose, und/oder insbesondere wobei der Füllstoff in der Zusammensetzung in einer Menge von 0,1 bis 50 Gcw.-%, insbesondere 0,2 bis 30 Gew.-%, vorzugsweise 0,5 bis 10 Gew.-%, bezogen auf die Zusammensetzung, vorliegt und/oder insbesondere wobei als Gleitmittel ein Fettsäuresalz zugegeben ist und/oder daß das Gleitmittel in der Zusammensetzung in einer Menge von 0,1 bis 50 Gew.-%, insbesondere 0,2 bis 30 Ges.-%, vorzugsweise 0,5 bis 10 Gew.-%, bezogen auf die Zusammensetzung, vorliegt und/oder insbesondere wobei das Sprengmittel ausgewählt ist aus der Gruppe von Stärke, Cellulose, insbesondere mikrokristalliner Cellulose, und Polyvinylpyrrolidon, vorzugsweise Croscarmellosenatrium, und/oder insbesondere wobei das Sprengmittel in der Zusammensetzung in einer Menge von 0,2 bis 20 Gew.-%, insbesondere 0,5 bis 15 Gew.-%, vorzugsweise 1 bis 10 Gew.-%, bezogen auf die Zusammensetzung, vorliegt.

10. Zusammensetzung, insbesondere Arzneimittel, nach einem der vorangehenden Ansprüche zür Anwendung in der propphylaktischen und/oder therapeutischen Behandlung von mit renalen Dysfunktionen, insbesondere Niereninsuffizienz, einhergehenden Erkrankungen und/oder Störungen, insbesondere Hyperphosphatämie und/oder Hypercalcämie.

11. Verwendung einer Kombination von Calciumacetat einerseits und Magnesiumsatz andererseits zur Herstellung einer Zusammensetzung, insbesondere eines Arzneimittels zur prophylaktischen und/oder therapeutischen Behandlung von mit renalen Dysfunktionen, insbesondere Niereninsuffizienz, einhergehenden Erkrankungen und/oder Störungen, insbesondere Hyperphosphatämie und/oder Hypercalcämie,
**dadurch gekennzeichnet,**
**daß** das Calciumacetat in Form eines mittels Wirbelschichtgranulation hergestellten Caicimacetatgranulales eingesetzt wird.

12. Verfahren zur Herstellung von partikulärem Calciumacetat,
**dadurch gekennzeichnet,**
**daß** das Calciumacetat mittels Wirbelschichtgranulation in Form von Calciumacetatgranulat hergestellt wird.

13. Partikuläres Calciumacetat, erhältlich in Form von Calciumacetatgranulat mittels Wirbelschichtgranulation.

14. Partikuläres Calciumacetat nach Anspruch 13, enthaltend außerdem mindestens einen weiteren Inhaltsstoff, insbesondere wobei der Inhaltsstoff ausgewählt ist aus der Gruppe von Verarbeitungs- und/oder Granulierhilfsmitteln, insbesondere Kohlenhydraten, Eiweißen oder deren Mischungen, vorzugsweise (Mais-)Stärke, Saccharose und/oder Gelatine.

15. Arzneimittel, insbesondere zur prophylaktischen und/oder therapeutischen Behandlung von mit renalen Dysfünktionen, insbesondere Niereninsuffizienz, einhergehenden Erkrankungen und/oder Störungen, wie Hyperphosphatämie und/oder Hypercalcämie, enthaltend partikuläres Calciumacetat nach einem der Ansprüche 13 oder 14 zusammen mit mindestens einem Exzipienten.

## Claims

1. A method for the production of a composition, in particular a pharmaceutical for the prophylactic and/or therapeutic treatment of diseases and/or disorders involving renal dysfunction, in particular kidney insufficiency, such as hyperphosphataemia and/or hypercalcaemia, wherein the composition comprises a combination of calcium acetate on one hand, and magnesium salt on the other hand,
**characterized in that**
the method comprises the following process steps:
(a) providing a particulate calcium acetate granulate, wherein the calcium acetate granulate is produced by means of fluidized-bed granulation;
(b) providing a particulate magnesium salt component;
(c) production of a mixture of the calcium acetate granulate provided under (a), and the magnesium salt component provided under (b); and
(d) subsequent processing of the mixture produced under (c) into an applicable formulation, preferably being perorally applicable, in particular in pharmaceutical form.

2. The method according to claim 1, **characterized in that** in step (a) the fluidized-bed granulation is carried out based on a solution and/or suspension containing the calcium acetate, in particular wherein for the production of the solution and/or suspension water is used as the dissolvent and/or suspending agent, in particular wherein the calcium acetate is added to the solution and/or the suspension at an amount of 30 to 90% by weight, in particular 40 to 80% by weight, preferably 45 to 75% by weight, based on the solution and/or suspension, and calculated as calcium acetate, and/or in particular wherein the solution and/or suspension containing the calcium acetate contains the water at an amount of 50 to 30% by weight, in particular 8 to 25% by weight, preferably 10 to 20% by weight, based on the solution and/or suspension.

3. The method according to claim 2, **characterized in that** at least one processing and/or granulating auxiliary agent, in particular at least one viscosity increasing substance, preferably based on protein, is added to the solution and/or suspension containing the calcium acetate, in particular at an amount of 0.1 to 3% by weight, in particular 0.2 to 2% by weight, preferably 0.3 to 1.5% by weight, based on the solution and/or suspension, in particular wherein at least one additional substance is added to the solution and/or suspension containing the calcium acetate, in particular of the group of processing and/or granulating auxiliary agents, in particular carbohydrates, preferably (corn) starch and/or saccharose, in particular at an amount of 0.5 to 30% by weight, in particular 1 to 25% by weight, preferably 3 to 20% by weight, based on the solution and/or suspension.

4. A composition, in particular pharmaceutical for the prophylactic and/or therapeutic treatment of diseases and/or disorders involving renal dysfunction, in particular kidney insufficiency, such as hyperphosphataemia and/or hypercalcaemia, wherein the composition comprises a combination of calcium acetate on one hand, and magnesium salt on the other hand,
**characterized in that**
the composition is obtained by means of a method according to one of the previous claims.

5. The composition, in particular pharmaceutical for the prophylactic and/or therapeutic treatment of diseases and/or disorders involving renal dysfunction, in particular kidney insufficiency, such as hyperphosphataemia and/or hypercalcaemia, wherein the composition comprises - each in effective, particularly pharmaceutically effective amounts, and together with at least one excipient - a combination of calcium acetate on one hand, and magnesium salt on the other hand,
**characterized in that**
the calcium acetate is used in the form of a calcium acetate granulate produced by means of fluidized-bed granulation.

6. The composition according to claims 4 or 5, **characterized in that** the composition comprises calcium acetate at an amount of 5 to 30% by weight, in particular 6 to 25% by weight, preferably 8 to 20% by weight, based on the composition, and calculated as calcium, and/or that the composition comprises calcium acetate per application unit, in particular per tablet, at an amount of 50 to 200 mg, in particular 75 to 150 mg, preferably 90 to 120 mg, preferably about 110 mg, calculated as calcium.

7. The composition according to claims 4 or 7, **characterized in that** the magnesium salt is a physiologically acceptable magnesium salt, preferably magnesium carbonate, and/or that the composition contains the magnesium slat, in particular magnesium carbonate, at an amount of 1 to 20% by weight, in particular 2 to 15% by weight, preferably 3 to 10% by weight, preferably 4 to 8% by weight, based on the composition, and calculated as magnesium, and/or that the composition comprises the magnesium salt, in particular magnesium carbonate, per application unit, in particular per tablet, at an amount of 20 to 100 mg, in particular 30 to 80 mg, preferably 40 to 70 mg, most preferably about 60 mg, calculated as magnesium.

8. The composition according to claims 4 or 7, **characterized in that** the composition comprises a calcium acetate/magnesium slat ratio, in particular a calcium acetate/magnesium carbonate ratio, calculated as calcium on one hand, and magnesium on the other hand, within the range of 10:1 to 1:5, in particular 5:1 to 1:2, preferably 4:1 to 1:1, preferably 2.5:1 to 1.2:1.

9. The composition according to claims 4 or 8, **characterized in that** the composition comprises at least one additional substance, in particular of the group of fillers, processing aids, lubricants, and disintegrants, in particular wherein the filler is selected from the group of lactose, sorbitol, and saccharose, preferably saccharose, and/or in particular wherein the filler in the composition is present at an amount of 0.1 to 50% by weight, in particular 0.2 to 30% by weight, preferably 0.5 to 10% by weight, based on the composition, and/or in particular wherein a fatty acid salt is added as the lubricant, and/or that the lubricant in the composition is present at an amount of 0.1 to 50% by weight, in particular 0.2 to 30% by weight, preferably 0.5 to 10% by weight, based on the composition, and/or in particular wherein the disintegrant is selected from the group of starch, cellulose, in particular microcrystalline cellulose, and polyvinyl pyrrolidone, preferably croscarmellosenatrium, and/or in particular wherein the disintegrant in the composition is present at an amount of 0.2 to 20% by weight, in particular 0.5 to 15% by weight, preferably 1 to 10% by weight, based on the composition.

10. The composition, in particular pharmaceutical according to one of the previous claims for use in the prophylactic and/or therapeutic treatment of diseases and/or disorders involving renal dysfunction, in particular kidney insufficiency, such as hyperphosphataemia and/or hypercalcaemia.

11. Use of a combination of calcium acetate on one hand, and magnesium slat on the other hand for the production of a composition, in particular of a pharmaceutical for the prophylactic and/or therapeutic treatment of diseases and/or disorders involving renal dysfunction, in particular kidney insufficiency, such as hyperphosphataemia and/or hypercalcaemia,
**characterized in that**
calcium acetate is used in the form of a calcium acetate granulate produced by means of fluidized-bed granulation.

12. A method for the production of particulate calcium acetate,
**characterized in that**
calcium acetate is produced in the form of calcium acetate granulate by means of fluidized-bed granulation.

13. A particulate calcium acetate, obtainable in the form of calcium acetate granulate by means of fluidized-bed granulation.

14. The particulate calcium acetate according to claim 13, further comprising at least one additional substance, in particular wherein the substance is selected from the group of processing and/or granulating auxiliary agents, in particular carbohydrates, egg white, or the mixtures thereof, preferably (corn) starch, saccharose, and/or gelatin.

15. A pharmaceutical, in particular for the prophylactic and/or therapeutic treatment of diseases and/or disorders involving renal dysfunction, in particular kidney insufficiency, such as hyperphosphataemia and/or hypercalcaemia, comprising particulate calcium acetate according to one of the claims 13 or 14, together with at least one excipient.

## Revendications

1. Procédé de préparation d'une composition, en particulier d'un médicament pour le traitement prophylactique et/ou thérapeutique d'affections et/ou de troubles comprenant des dysfonctions rénales, en particulier une insuffisance rénale, tels une hyperphosphatémie et/ou une hypercalcémie, la composition comportant une combinaison d'acétate de calcium d'une part et de sels de magnésium d'autre part,
**caractérisé en ce que**
le procédé comprend les étapes suivantes :
(a) mise à disposition de granulés d'acétate de calcium en particules, les granulés d'acétate de calcium étant préparés par granulation en lit fluidisé ;
(b) mise à disposition d'un composant de sel de magnésium en particules ;
(c) préparation d'un mélange des granulés d'acétate de calcium préparés sous (a) et du composant de sel de magnésium préparé sous (b) ; et
(d) ensuite, transformation du mélange préparé sous (c) en une formulation administrable, de préférence administrable oralement, en particulier en une forme de médicament.

2. Procédé selon la revendication 1, **caractérisé en ce que** dans l'étape (a), la granulation en lit fluidisé est effectuée en partant d'une solution et/ou d'une suspension contenant l'acétate de calcium, en particulier **en ce que** de l'eau est utilisée pour la préparation de la solution et/ou de la suspension, en particulier **en ce que** l'acétate de calcium est ajouté à la solution et/ou à la suspension en une quantité de 30 à 90 % en poids, en particulier de 40 à 80 % en poids, de préférence de 45 à 75 % en poids, rapportée à la solution et/ou à la suspension et calculée comme acétate de calcium, et/ou en particulier **en ce que** la solution et/ou la suspension contenant l'acétate de calcium contient l'eau en une quantité de 5 à 30 % en poids, en particulier de 8 à 25 % en poids, de préférence de 10 à 20 % en poids, rapportée à la solution et/ou à la suspension.

3. Procédé selon la revendication 2, **caractérisé en ce que** l'on ajoute à la solution et/ou à la suspension contenant l'acétate de calcium au moins un auxiliaire de transformation et/ou un auxiliaire de granulation, en particulier au moins une substance accroissant la viscosité, de préférence à base de protéine, de préférence de la gélatine, en particulier en une quantité de 0,1 à 3 % en poids, en particulier de 0,2 à 2 % en poids, de préférence de 0,3 à 1,5 % en poids, rapportée à la solution et/ou à la suspension, en particulier **en ce que** l'on ajoute à la solution et/ou à la suspension contenant l'acétate de calcium un composant supplémentaire, en particulier du groupe des auxiliaires de transformation et/ou de granulation, en particulier des hydrates de carbone, de préférence de l'amidon (de maïs) et/ou du saccharose, en particulier en une quantité de 0,5 à 30 % en poids, en particulier de 1 à 25 % en poids, de préférence de 3 à 20 % en poids, rapportée à la solution et/ou à la suspension.

4. Composition, en particulier médicament pour le traitement prophylactique et/ou thérapeutique d'affections et/ou de troubles comprenant des dysfonctions rénales, en particulier une insuffisance rénale, tels une hyperphosphatémie et/ou une hypercalcémie, la composition comportant une combinaison d'acétate de calcium d'une part et de sels de magnésium d'autre part,
**caractérisée en ce que**
la composition peut être obtenue par un procédé selon l'une quelconque des revendications précédentes.

5. Composition, en particulier médicament pour le traitement prophylactique et/ou thérapeutique d'affections ou de troubles comprenant des dysfonctions rénales, en particulier une insuffisance rénale, tels une hyperphosphatémie et/ou une hypercalcémie, la composition comportant - dans des quantités respectivement efficaces, en particulier pharmaceutiquement efficaces et ensemble avec au moins un excipient - une combinaison d'acétate de calcium d'une part et de sels de magnésium d'autre part,
**caractérisée en ce que**
l'acétate de calcium est utilisé sous la forme d'un granulé de calcium fabriqué au moyen de granulation en lit fluidisé.

6. Composition selon la revendication 4 ou 5, **caractérisée en ce que** la composition contient l'acétate de calcium en une quantité de 5 à 30 % en poids, en particulier de 6 à 25 % en poids, de préférence de 8 à 20 % en poids, rapportée à la composition et calculée comme calcium, et/ou **en ce que** la composition comporte par unité d'application, en particulier par comprimé, l'acétate de calcium en une quantité de 50 à 200 mg, en particulier de 75 à 150 mg, de préférence de 90 à 120 mg, encore plus préférablement d'environ 110 mg, calculée comme calcium.

7. Composition selon l'une quelconque des revendications 4 à 7, **caractérisée en ce que** le sel de magnésium est un sel de magnésium physiologiquement acceptable, de préférence du carbonate de magnésium, et/ou **en ce que** la composition contient le sel de magnésium, en particulier le carbonate de magnésium, en une quantité de 1 à 20 % en poids, en particulier de 2 à 15 % en poids, de préférence de 3 à 10 % en poids, encore plus préférablement de 4 à 8 % en poids, rapportée à la composition et calculée comme magnésium, et/ou **en ce que** la composition comporte le sel de magnésium, en particulier le carbonate de magnésium, en une quantité de 20 à 100 mg, en particulier de 30 à 80 mg, de préférence de 40 à 70 mg, encore plus préférablement d'environ 60 mg, calculée comme magnésium, par unité d'application, en particulier par comprimé.

8. Composition selon l'une quelconque des revendications 4 à 7, **caractérisée en ce que** la composition présente un rapport acétate de calcium/sel de magnésium, en particulier un rapport acétate de calcium/carbonate de magnésium, calculé comme calcium d'un côté et comme magnésium de l'autre côté, dans l'intervalle entre 10 : 1 et 1 : 5, en particulier entre 5 : 1 et 1 : 2, de préférence entre 4 : 1 et 1 : 1, encore plus préférablement entre 2,5 : 1 et 1,2 : 1.

9. Composition selon l'une quelconque des revendications 4 à 8, **caractérisée en ce que** la composition comporte au moins un autre constituant, en particulier du groupe des charges, des auxiliaires de transformation, des lubrifiants et des agents dispersants, la charge étant choisie en particulier parmi le groupe du lactose, du sorbitol et du saccharose, le saccharose étant préféré, et/ou la charge étant présente en particulier dans la composition en une quantité de 0,1 à 50 % en poids, en particulier de 0,2 à 30 % en poids, de préférence de 0,5 à 10 % en poids, rapportée à la composition, et/ou un sel d'un acide gras étant ajouté en particulier comme lubrifiant, et/ou le lubrifiant étant présent dans la composition en une quantité de 0,1 à 50 % en poids, en particulier de 0,2 à 30 % en poids, de préférence de 0,5 à 10 % en poids, rapportée à la composition, et/ou l'agent dispersant étant choisi en particulier parmi le groupe de l'amidon, de la cellulose, en particulier de la cellulose microcristalline, et de la polyvinylpyrrolidone, de préférence le croscarmellose de sodium, et/ou l'agent dispersant étant présent en particulier dans la composition en une quantité de 0,2 à 20 % en poids, en particulier de 0,5 à 15 % en poids, de préférence de 1 à 10 % en poids, rapportée à la composition.

10. Composition, en particulier médicament selon l'une quelconque des revendications précédentes, pour l'application dans le traitement prophylactique et/ou thérapeutique d'affections ou de troubles comprenant des dysfonctions rénales, en particulier une insuffisance rénale, tels une hyperphosphatémie et/ou une hypercalcémie.

11. Utilisation d'une combinaison d'acétate de calcium d'une part et d'un sel de magnésium d'autre part à la préparation d'une composition, en particulier d'un médicament pour le traitement prophylactique et/ou thérapeutique d'affections ou de troubles comprenant des dysfonctions rénales, en particulier une insuffisance rénale, tels une hyperphosphatémie et/ou une hypercalcémie,
**caractérisée en ce que**
l'acétate de calcium est utilisé sous la forme d'un granulé d'acétate de calcium préparé par granulation en lit fluidisé.

12. Procédé de préparation d'acétate de calcium en particules,
**caractérisé en ce que**
l'acétate de calcium est préparé par granulation en lit fluidisé sous la forme de granulés d'acétate de calcium.

13. Acétate de calcium en particules, pouvant être obtenu par granulation en lit fluidisé sous la forme de granulés d'acétate de calcium.

14. Acétate de calcium en particules selon la revendication 13, contenant en outre au moins un autre constituant, le constituant étant choisi en particulier parmi le groupe des auxiliaires de transformation et/ou de granulation, en particulier des hydrates de carbone, des protéines ou de leurs mélanges, de préférence de l'amidon (de mais), du saccharose et/ou de la gélatine.

15. Médicament pour le traitement prophylactique et/ou thérapeutique d'affections ou de troubles comprenant des dysfonctions rénales, en particulier une insuffisance rénale, tels une hyperphosphatémie et/ou une hypercalcémie, contenant de l'acétate de calcium en particules selon l'une quelconque des revendications 13 ou 14; ensemble avec au moins un excipient.
